# EUROPEAN PATENT APPLICATION

(11) **EP 4 477 697 A1**
(43) Date of publication of application: **18.12.2024**
(21) Application number: 23752845.0
(22) Date of filing: 06.02.2023
(51) Int. Cl.: C08J 11/16, C08J 11/12

(54) **METHOD FOR DECOMPOSING CROSSLINKED RUBBER**

(30) Priority: 08.02.2022 JP 2022018306
(71) Applicant: BRIDGESTONE CORPORATION, Chuo-ku Tokyo 104-8340 (JP); Tohoku University, Sendai-shi, Miyagi 980-8577 (JP); National Institute of Advanced Industrial Science and Technology, Chiyoda-ku Tokyo 100-8921 (JP)
(72) Inventor: HOJO, Masahiro, Tokyo 104-8340 (JP); OKUNO, Akira, Tokyo 104-8340 (JP); KUNO, Marino, Tokyo 104-8340 (JP); HOMMA, Masahiro, Tokyo 104-8340 (JP); YOSHIOKA, Toshiaki, Sendai-shi, Miyagi 980-8577 (JP); KUMAGAI, Shogo, Sendai-shi, Miyagi 980-8577 (JP); FUKAYA, Norihisa, Tsukuba-shi, Ibaraki 305-8561 (JP); WAHYU, Satpriyo Putro, Tsukuba-shi, Ibaraki 305-8561 (JP); CHOI, Jun-Chul, Tsukuba-shi, Ibaraki 305-8561 (JP); MIFTAH, Faried, Tsukuba-shi, Ibaraki 305-8561 (JP); YAMASHITA, Hiroshi, Tsukuba-shi, Ibaraki 305-8561 (JP); FUJITANI, Tadahiro, Tsukuba-shi, Ibaraki 305-8561 (JP)
(74) Representative: Marks & Clerk LLP
(86) International application number: PCT/JP2023/003876
(87) International publication number: WO 2023/153381

(57) **Abstract**

The present disclosure addresses the problem of providing a method for decomposing a crosslinked rubber that can improve monomer yield. The solution is a method of decomposing a crosslinked rubber that includes: a first decomposition step of pyrolyzing a crosslinked rubber containing a diene rubber at a temperature of 150°C or more and 400°C or less; and a second decomposition step of pyrolyzing a decomposition product obtained by the first decomposition step under an inert gas atmosphere and in the presence of a catalyst at a temperature of 300°C or more and 950°C or less. Preferably 80 mass% or more of the diene rubber in the crosslinked rubber is decomposed to diene oligomers having a weight-average molecular weight of 100 to 50,000 via the first decomposition step.

## Description

### TECHNICAL FIELD

The present disclosure relates to a method of decomposing a crosslinked rubber.

### BACKGROUND

Conventionally, rubber products made primarily from crosslinked rubber such as vulcanized rubber are difficult to reuse and are often reused as fuel after the life of a product, particularly in cement plants and the like. However, in recent years, with the growing concern about environmental issues, there is a demand for development of methods to reuse materials obtained by decomposing rubber products instead of burning rubber products as fuel.

There are various methods for decomposing a crosslinked rubber. For example, a technique of pyrolysis of crosslinked rubber at high temperatures is known. Further, Patent Literature (PTL) 1 describes a method of decomposing polyisoprene rubber by microorganisms.

### CITATION LIST

### Patent Literature

PTL 1: JP 2009-247241 A

### SUMMARY

### (Technical Problem)

As mentioned above, there are various methods for decomposing a crosslinked rubber. From the viewpoint of further improving the recyclability of crosslinked rubber, it is important to increase the yield of monomers obtained through decomposition.

However, when crosslinked rubber is pyrolyzed at high temperature as described above, decomposition products gasify or aromatize, resulting in lower yields of monomers. Further, when using microorganisms to decompose crosslinked rubber, as in the technology described in PTL 1, a long time is required for decomposition, and further, the yield of monomers is low.

It would be helpful to solve the problems of the conventional technologies described above and to provide a method of decomposing a crosslinked rubber that can improve the yield of monomers.

### (Solution to Problem)

Primary features of a method of decomposing a crosslinked rubber according to the present disclosure, as a solution to the problems described above, are as follows.
[1] A method of decomposing a crosslinked rubber, the method comprising:
   a first decomposition step of pyrolyzing a crosslinked rubber containing a diene rubber at a temperature of 150°C or more and 400°C or less; and
   a second decomposition step of pyrolyzing a decomposition product obtained by the first decomposition step under an inert gas atmosphere and in the presence of a catalyst at a temperature of 300°C or more and 950°C or less.
[2] The method of decomposing a crosslinked rubber according to [1], wherein 80 mass% or more of the diene rubber in the crosslinked rubber is decomposed to diene oligomers having a weight-average molecular weight of 100 to 50,000 via the first decomposition step.
[3] The method of decomposing a crosslinked rubber according to [2], wherein 30 mass% or more of the diene oligomers are decomposed to hydrocarbons having 12 or less carbon atoms via the second decomposition step.
[4] The method of decomposing a crosslinked rubber according to any one of [1] to [3], wherein 20 mass% or more of the diene rubber in the crosslinked rubber is decomposed to hydrocarbons having 12 or less carbon atoms via the first decomposition step and the second decomposition step.
[5] The method of decomposing a crosslinked rubber according to any one of [1] to [4], wherein the diene rubber includes at least one rubber selected from the group consisting of isoprene skeleton rubber, styrene-butadiene rubber, and butadiene rubber.
[6] The method of decomposing a crosslinked rubber according to any one of [1] to [5], wherein the second decomposition step is performed in the presence of at least one basic catalyst selected from the group consisting of TiO₂, ZrO₂, MgO, La₂O₃, CeO₂, Y₂O₃, Li₂CO₃, Na₂CO₃, Rb₂CO₃, and Cs₂CO₃.
[7] The method of decomposing a crosslinked rubber according to any one of [1] to [6], wherein the crosslinked rubber further contains carbon black.
[8] The method of decomposing a crosslinked rubber according to any one of [1] to [7], wherein the crosslinked rubber further contains sulfur.

### (Advantageous Effect)

The present disclosure provides a method of decomposing a crosslinked rubber that can improve the yield of monomers.

### BRIEF DESCRIPTION OF THE DRAWINGS

In the accompanying drawings:

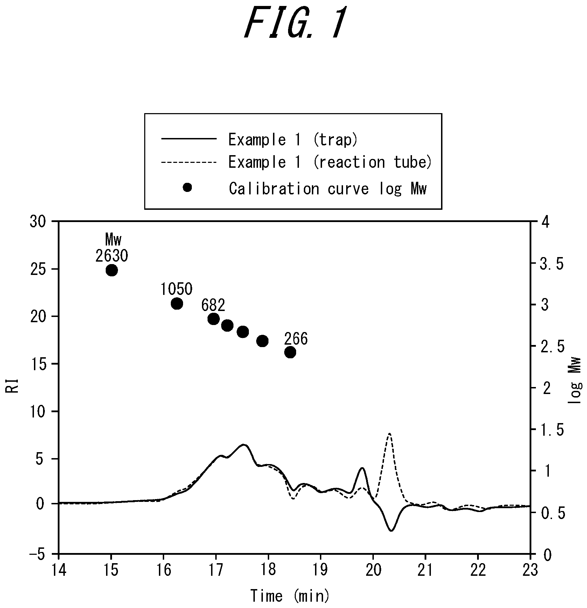

FIG. 1 is a GPC chart of oligomers recovered in Example 1.

### DETAILED DESCRIPTION

The following is a detailed illustrative description of a method of decomposing a crosslinked rubber based on an embodiment of the present disclosure.

The method of decomposing a crosslinked rubber includes:
a first decomposition step of pyrolyzing a crosslinked rubber containing a diene rubber at a temperature of 150°C or more and 400°C or less; and
a second decomposition step of pyrolyzing a decomposition product obtained by the first decomposition step under an inert gas atmosphere and in the presence of a catalyst at a temperature of 300°C or more and 950°C or less.

According to the method of decomposing a crosslinked rubber, pyrolyzing at a relatively low temperature of 150°C or more and 400°C or less in the first decomposition step can better suppress gasification and aromatization of decomposition products and improve the retention rate of a monomer skeleton (isoprene skeleton, butadiene skeleton, and the like) in the diene rubber than normal high temperature pyrolysis.

Further, in the method of decomposing a crosslinked rubber, in the second decomposition step, decomposition product obtained in the first decomposition step is pyrolyzed under an inert gas atmosphere and in the presence of a catalyst at a temperature of 300°C or more and 950°C or less, thereby inhibiting hydrogenation of double bonds in monomer skeletons and oxidation, so that the decomposition product (intermediate decomposition product) can be decomposed into monomers (in particular, diene monomers).

Accordingly, the method of decomposing a crosslinked rubber may improve the yield of monomers ultimately obtained from the crosslinked rubber.

### <First decomposition step>

The method of decomposing a crosslinked rubber according to the present embodiment includes a first decomposition step of pyrolyzing a crosslinked rubber containing a diene rubber at a temperature of 150°C or more and 400°C or less.

### (Crosslinked rubber)

The crosslinked rubber that is the decomposition target of the method according to the present embodiment contains a diene rubber and may further contain carbon black, sulfur, and the like.

The form of the crosslinked rubber is not particularly limited and may be, for example, powdered rubber. Rubber powder can be obtained by shredding and granulating used rubber products such as waste tires. A granulating step may include multiple steps, such as a preliminary granulating step and a fine granulating step, or particle size of powdered rubber used may be adjusted after the granulating step via a sorting step.

### -- Diene rubber --

The diene rubber is a rubber containing units derived from diene monomers (diene units) and may further contain units derived from copolymerizable comonomers.

The diene monomer-derived units described above enable crosslinking (vulcanization) of diene rubber and also enable diene rubber to develop rubber-like elongation and strength. In crosslinked rubber, diene rubber is normally present in a crosslinked state, but some diene rubber may be not crosslinked. Diene monomers (diene compounds) specifically include 1,3-butadiene, isoprene, 1,3-pentadiene, 2,3-dimethyl-1,3-butadiene, and the like.

On the other hand, aromatic vinyl compounds and the like are examples of copolymerizable comonomers. Examples of aromatic vinyl compounds include styrene, o-methylstyrene, m-methylstyrene, p-methylstyrene, o,p-dimethylstyrene, o-ethylstyrene, m-ethylstyrene, p-ethylstyrene, and the like.

Further, examples of the diene rubber include isoprene skeleton rubber, styrene-butadiene rubber (SBR), butadiene rubber (BR), and chloroprene rubber (CR). Isoprene skeleton rubber is rubber having an isoprene unit as the main skeleton, such as natural rubber (NR) and synthetic isoprene rubber (IR). Among these, the diene rubber preferably includes at least one selected from the group consisting of isoprene skeleton rubber, styrene-butadiene rubber, and butadiene rubber. When the diene rubber includes at least one selected from the group consisting of isoprene skeleton rubber, styrene-butadiene rubber, and butadiene rubber, diene monomers such as isoprene and butadiene that are easily reused are obtainable.

The content of the diene rubber in the crosslinked rubber is not particularly limited and is preferably, for example, in a range from 10 mass% to 100 mass%. From the viewpoint of further improving the yield of butadiene and isoprene, the content of the diene rubber in the crosslinked rubber is more preferably in a range from 30 mass% to 100 mass%.

### -- Carbon Black --

The crosslinked rubber may further contain carbon black. The method of decomposing a crosslinked rubber can decompose diene rubber in the crosslinked rubber in the first decomposition step to a lower molecular weight, for example, to liquid polymers and liquid oligomers. Therefore, even when the crosslinked rubber contains carbon black, the carbon black can be easily separated and recovered after the first decomposition step by, for example, solid-liquid separation. By recovering carbon black prior to pyrolysis in the second decomposition step, degradation of carbon black can be suppressed and reuse as high-grade carbon black is possible. Further, even when the crosslinked rubber contains carbon black, the crosslinked rubber may be efficiently decomposed.

The content of carbon black in the crosslinked rubber is not particularly limited and may be, for example, in a range from 10 parts by mass to 150 parts by mass, and preferably in a range from 30 parts by mass to 120 parts by mass, relative to 100 parts by mass of the diene rubber.

### -- Sulfur --

The crosslinked rubber may further contain sulfur. In crosslinked rubber, sulfur is typically present in a form crosslinking diene rubber (as a crosslink for diene rubber), but some sulfur may be free. The method of decomposing a crosslinked rubber can decompose diene rubber in the crosslinked rubber in the first decomposition step to a lower molecular weight, for example, to liquid polymers and liquid oligomers. Therefore, even when the crosslinked rubber contains sulfur, the sulfur can be easily recovered after the first decomposition step by, for example, solid-liquid separation. Recovery of sulfur prior to pyrolysis in the second decomposition step allows the sulfur to be reused. Further, even when a catalyst is used in the second decomposition step, recovery may prevent sulfur from becoming a catalyst poison or from re-crosslinking (revulcanizing) a decomposition product. Accordingly, even when the crosslinked rubber contains sulfur, the crosslinked rubber can be efficiently decomposed.

The content of sulfur in the crosslinked rubber is not particularly limited and may be, for example, in a range from 0.1 parts by mass to 10 parts by mass, and preferably in a range from 1 part by mass to 5 parts by mass, relative to 100 parts by mass of the diene rubber.

### -- Other components --

Aside from diene rubber, carbon black, and sulfur mentioned above, the crosslinked rubber may contain various components normally used in the rubber industry, such as a rubber component other than diene rubber, a filler other than carbon black (silica, calcium carbonate, and the like), a silane coupling agent, an antioxidant, a softener, a processing aid, a resin, a surfactant, an organic acid (stearic acid and the like), zinc oxide (zinc flower), a vulcanization accelerator, a crosslinking agent other than sulfur (peroxide and the like), and the like.

### (Reaction conditions and the like)

The first decomposition step is performed at a temperature of 150°C or more and 400°C or less. By performing the first decomposition step at 150°C or more, the speed of the decomposition reaction of the diene rubber in the crosslinked rubber is improved, and by performing the first decomposition step at 400°C or less, gasification and aromatization of decomposition product can be suppressed, and after decomposition, retention rate (selectivity) of monomer skeletons of the diene rubber in the crosslinked rubber is improved. Further, for example, when the crosslinked rubber contains carbon black, recovery and reuse as high-grade carbon black is possible. From the viewpoint of improving the decomposition reaction rate of the diene rubber, the first decomposition step is preferably performed at a temperature of 175°C or more, and more preferably at 190°C or more. Further, from the viewpoint of improving selectivity for product retaining a monomer skeleton, 350°C or less is preferable, and 300°C or less is more preferable.

The first decomposition step is preferably performed under an inert gas atmosphere. By performing the first decomposition step under an inert gas atmosphere, oxidation and reduction of a decomposition product can be suppressed, in particular hydrogenation of double bonds in oligomers and monomers in the decomposition product. Further, when the crosslinked rubber contains carbon black, oxidation of the carbon black can also be suppressed. Examples of inert gases include nitrogen, carbon dioxide, argon, helium, and the like.

To perform the first decomposition step under an inert gas atmosphere, for example, when a batch reactor is used, the atmosphere charged into the reactor may be an inert gas, and when a flow reactor is used, the atmosphere circulated through the reactor may be an inert gas. Although hydrogen may be produced during the first decomposition step, the atmosphere of the first decomposition step does not take into account hydrogen produced.

The first decomposition step may be performed at any pressure, and may be performed at reduced, normal, or increased pressure, and is preferably performed under reduced pressure or normal pressure. As an example, reaction pressure of the first decomposition step is preferably 1000 kPa to 65 kPa. By performing the first decomposition step under reduced pressure or normal pressure, polymerization (repolymerization) of oligomers and monomers in decomposition product may be suppressed.

The reaction time of the first decomposition step is not particularly limited. As an example, the reaction time for the first decomposition step is preferably from 1 min to 180 min, more preferably 3 min to 60 min, and even more preferably 5 min to 30 min.

The first decomposition step may or may not use a catalyst, and use of no catalyst is preferred. The use of no catalyst in the first decomposition step reduces costs. When using a catalyst, any catalyst that promotes the decomposition reaction of crosslinked rubber may be used.

### (Decomposition product (intermediate decomposition product))

In the method of decomposing a crosslinked rubber according to the present embodiment, preferably 80 mass% or more of the diene rubber in the crosslinked rubber is decomposed to diene oligomers having a weight-average molecular weight of 100 to 50,000 via the first decomposition step. By decomposing 80 mass% or more of diene rubber in the crosslinked rubber to diene oligomers having a weight-average molecular weight of 100 to 50,000 via the first decomposition step, the yield of diene monomers via the second decomposition step described below can be further improved. From the viewpoint of the yield of diene monomers after the second decomposition step, more preferably 85 mass% or more of diene rubber in the crosslinked rubber is decomposed to diene oligomers having a weight-average molecular weight of 100 to 50,000 via the first decomposition step.

Hereinafter, oligomers are defined as having a weight-average molecular weight of 50,000 or less and containing two or more monomer units. Further, diene oligomers are oligomers that contain two or more units derived from diene monomers (diene units). Further, weight-average molecular weight (Mw) may be measured by gel permeation chromatography (GPC).

### <Second decomposition step>

The method of decomposing a crosslinked rubber according to the present embodiment includes a second decomposition step of pyrolyzing a decomposition product obtained by the first decomposition step under an inert gas atmosphere and in the presence of a catalyst at a temperature of 300°C or more and 950°C or less.

### (Reaction conditions and the like)

Performing the second decomposition step at 300°C or more increases the rate of a decomposition reaction of the decomposition product obtained by the first decomposition step, and performing the second decomposition step at 950°C or less improves selectivity for a product that retains a monomer skeleton. The second decomposition step is more preferably performed at 500°C or more from the viewpoint of improving the decomposition reaction rate, and more preferably performed at 900°C or less from the viewpoint of improving selectivity for a product that retains a monomer skeleton.

The second decomposition step is performed under an inert gas atmosphere. By performing the second decomposition step under an inert gas atmosphere, oxidation and reduction of a decomposition product can be suppressed, in particular hydrogenation of double bonds in monomers in the decomposition product. Examples of inert gases include nitrogen, carbon dioxide, argon, helium, and the like.

To perform the second decomposition step under an inert gas atmosphere, for example, when a batch reactor is used, the atmosphere charged into the reactor may be an inert gas, and when a flow reactor is used, the atmosphere circulated through the reactor may be an inert gas. Although hydrogen may be produced during the second decomposition step, the atmosphere of the second decomposition step does not take into account hydrogen produced.

The second decomposition step may, for example, when performed under pressure, pyrolyze a decomposition product obtained by the first decomposition step in a solvent. Any solvent that does not inhibit the decomposition reaction may be used as the solvent, such as ethers, an aliphatic hydrocarbon, an alicyclic hydrocarbon, an aromatic hydrocarbon (aromatic solvent), and the like. More specifically, tetrahydrofuran (THF), hexane, cyclohexane, pentane, cyclopentane, toluene, and xylene are preferred solvents, and toluene and tetrahydrofuran are even more preferred.

The amount of solvent used is preferably 10 mL or more, more preferably 50 mL or more, and preferably 500 mL or less, more preferably 200 mL or less per 1 g of decomposition product obtained by the first decomposition step (or intermediate product for the second decomposition step). When the amount of solvent used is 10 mL or more per 1 g of decomposition product obtained by the first decomposition step (or intermediate product for the second decomposition step), the decomposition reaction progresses further. In terms of cost, the amount of solvent used is preferably 500 mL or less per 1 g of decomposition product obtained by the first decomposition step (or intermediate product for the second decomposition step).

### (Catalyst)

The second decomposition step is performed in the presence of a catalyst. The catalyst may be an acidic, neutral, or basic catalyst, and among these, a basic catalyst is preferred. Further, the second decomposition step is more preferably performed in the presence of at least one basic catalyst selected from the group consisting of TiO₂, ZrO₂, MgO, La₂O₃, CeO₂, Y₂O₃, Li₂CO₃, Na₂CO₃, Rb₂CO₃, and Cs₂CO₃. Performing the second decomposition step in the presence of at least one of these basic catalysts increases the rate of decomposition reaction (depolymerization reaction) of decomposition product obtained by the first decomposition step, and also increases selectivity for a product that retains a monomer skeleton. One such catalyst may be used alone, or two or more catalysts may be used in combination.

The amount of catalyst used is preferably 1 part by mass or more, more preferably 10 parts by mass or more, even more preferably 100 parts by mass or more, and preferably 8000 parts by mass or less, more preferably 4000 parts by mass or less, even more preferably 500 parts by mass or less, relative to 100 parts by mass of decomposition product obtained by the first decomposition step (or intermediate product for the second decomposition step). When the amount of catalyst used is 100 parts by mass or more per 100 parts by mass of decomposition product obtained by the first decomposition step (or intermediate product for the second decomposition step), the decomposition reaction progresses further. In terms of cost, the amount of catalyst used is preferably 500 parts by mass or less per 100 parts by mass of decomposition product obtained by the first decomposition step (or intermediate product for the second decomposition step).

### (Decomposition product)

In the method of decomposing a crosslinked rubber according to the present embodiment, the second decomposition step preferably decomposes 20 mass% or more of diene oligomer having a weight-average molecular weight of 100 to 50,000 obtained via the first decomposition step to hydrocarbons having 12 or less carbon atoms. Increased yield of hydrocarbons having 12 or less carbon atoms increases yield of reusable monomers and oligomers, further improving economic and environmental value of the decomposition method. From the viewpoint of yield of reusable monomers and oligomers, the second decomposition step more preferably decomposes 30 mass% or more of diene oligomer having a weight-average molecular weight of 100 to 50,000 obtained via the first decomposition step to hydrocarbons having 12 or less carbon atoms.

Further, in the method of decomposing a crosslinked rubber according to the present embodiment, preferably 0.1 mass% or more of diene rubber in the crosslinked rubber is decomposed to hydrocarbons having 12 or less carbon atoms via the first decomposition step and the second decomposition step. Increased yield of hydrocarbons having 12 or less carbon atoms increases yield of reusable monomers, further improving economic and environmental value of the decomposition method. From the viewpoint of yield of reusable monomers, more preferably 20 mass% or more of diene rubber in the crosslinked rubber is decomposed to hydrocarbons having 12 or less carbon atoms via the first decomposition step and the second decomposition step.

As a decomposition product, hydrocarbons having 12 or less carbon atoms vary depending on the type of diene rubber in the crosslinked rubber to be decomposed, and examples include 1,3-butadiene, isoprene, 1,3-pentadiene, 2,3-dimethyl-1,3-butadiene, limonene, and the like.

### <Other features>

In addition to the first decomposition step and the second decomposition step described above, the method of decomposing a crosslinked rubber according to the present embodiment may include another step. Examples of such a step include a crosslinked rubber pretreatment step (for example, shredding and granulating steps), and the like.

When the crosslinked rubber contains carbon black, a step of recovering carbon black from a decomposition product (intermediate decomposition product) is preferably included between the first decomposition step and the second decomposition step.

Further, the method of decomposing a crosslinked rubber according to the present embodiment may be performed in a batch reactor or in a flow reactor.

Further, decomposition products after a decomposition reaction may be separated and recovered by filtration, distillation, and the like, recovered by precipitation using poor solvents, and the like, and may be reused. Further, diene monomers ultimately obtained from the crosslinked rubber may be reused as raw materials for diene rubber (polymers).

### EXAMPLES

This disclosure is described in more detail below with reference to Examples. The present disclosure is not limited in any way by the Examples.

### <Weight-average molecular weight (Mw)>

Polystyrene-equivalent weight-average molecular weight (Mw) of decomposition products (intermediate decomposition products) was determined by gel permeation chromatography (GPC) using monodisperse polystyrene as a standard [GPC: HLC-8321 GPC/HT, produced by Tosoh Corporation, column: 2 × HT-806M, produced by Showa Denko K.K., detector: differential refractometer (RI)]. The measurement temperature was 40°C.

### <Preparation of crosslinked rubber samples>

A rubber composition was prepared by blending, relative to 100.1 parts by mass of natural rubber, 50.0 parts by mass of carbon black, 1.0 part by mass of antioxidant 6PPD [N-phenyl-N'-(1,3-dimethylbutyl)-p-phenylenediamine], 2.0 parts by mass of stearic acid, 2.5 parts by mass of zinc oxide, 1.5 parts by mass of vulcanization accelerator (N-cyclohexyl-2-benzothiazolesulfenamide), and 4.5 parts by mass of sulfur, and the rubber composition was heated and crosslinked to prepare crosslinked rubber. The resulting crosslinked rubber samples were shredded so that each side was approximately 2 mm to 6 mm.

### <First decomposition step>

### (Example 1)

Quartz wool was filled in the lower part of the reaction zone of a reaction tube provided with a sample inlet and a nitrogen introduction line at the upstream side. An ice-cooled trap, a first liquid nitrogen trap, and a second liquid nitrogen trap were disposed downstream of the reaction tube, and gas that passed through the second liquid nitrogen trap was collected in a gasbag.

A 10 g crosslinked rubber sample obtained as described above was fed into the reaction tube at a feed rate of 1 g/10 min. A pyrolysis reaction (first decomposition step) was performed at 330°C under a condition of nitrogen flow rate: 600 mL/min. Here, the supplied sample was placed on top of the quartz wool and undergoes a pyrolysis reaction.

Ten minutes after the sample feed was completed, the reaction tube was cooled and highly viscous material (oligomer) precipitated in the reaction tube and trap was directly recovered. The oligomer recovered was dark brown, highly viscous, and the amount recovered was 2.4 g. The molecular weight of the oligomers recovered was determined by GPC analysis. The respective results for oligomers precipitated in the reaction tube and in the trap are illustrated in FIG 1. When measured by liquid chromatography in accordance with test standard IP391, the ratio of the mass of aromatic compounds derived from natural rubber in the decomposition product (oligomers) to the mass of natural rubber in the crosslinked rubber sample was 25 mass%.

Further, at the same time, carbon black derived from the crosslinked rubber sample was recovered. The amount of carbon black recovered was 6.1 g.

### <Examples of second decomposition step>

### (Example 2)

A portion, 3.616 mg, of the oligomers obtained in the first decomposition step (oligomers obtained in Example 1) was dissolved in 0.72 mL of tetrahydrofuran. A portion, 5.0 µL, of the solution was impregnated with 1.162 mg of La₂O₃ catalyst, and the solvent was removed under reduced pressure. The La₂O₃ with 0.025 mg of the oligomers of the first decomposition step attached, obtained after solvent distillation, was heated to 650°C using a pyrolyzer (PY-3030D, produced by Frontier Laboratories Ltd.). The pyrolysis products were analyzed using a gas chromatograph-mass spectrometer and gas chromatography system (GCMS-QP2010 Plus and GC-2014, produced by Shimadzu Corporation, respectively, both with capillary columns Ultra ALLOY^{®} UA-1 (Ultra ALLOY is a registered trademark in Japan, other countries, or both), produced by Frontier Laboratories, Ltd). Analysis results confirmed that isoprene, limonene, hydrocarbons having 2 to 4 carbon atoms, and hydrocarbons having 5 to 10 carbon atoms (other than isoprene and limonene) were produced in yields of 8%, 0.5%, 4%, and 12%, respectively.

### (Example 3)

The pyrolysis reaction and analysis were performed as per Example 2, except that 0.939 mg of ZrO₂ was used as the catalyst instead of 1.162 mg of La₂O₃. The results confirmed that isoprene, limonene, hydrocarbons having 2 to 4 carbon atoms, and hydrocarbons having 5 to 10 carbon atoms (other than isoprene and limonene) were produced in yields of 7%, 0.4%, 4%, and 14%, respectively.

### (Example 4)

The pyrolysis reaction and analysis were performed as per Example 2, except that 0.913 mg of La₂O₃ was used as the catalyst instead of 1.162 mg of La₂O₃ and the temperature of the pyrolysis reaction was 850°C. The results confirmed that isoprene, limonene, hydrocarbons having 2 to 4 carbon atoms, and hydrocarbons having 5 to 10 carbon atoms (other than isoprene and limonene) were produced in yields of 11%, 0.3%, 28%, and 25%, respectively.

### (Example 5)

The pyrolysis reaction and analysis were performed as per Example 2, except that 0.929 mg of ZrO₂ was used as the catalyst instead of 1.162 mg of La₂O₃ and the temperature of the pyrolysis reaction was 850°C. The results confirmed that isoprene, limonene, hydrocarbons having 2 to 4 carbon atoms, and hydrocarbons having 5 to 10 carbon atoms (other than isoprene and limonene) were produced in yields of 10%, 0.4%, 30%, and 27%, respectively.

### (Examples 6 to 13)

Next, Examples 6 to 13 clarify conditions for obtaining higher amounts of isoprene and limonene.

### <First decomposition step>

1.5 g of vulcanized rubber sample shredded into 2 mm squares was filled into an alumina boat and placed in an electric furnace (ROP-001PG) chamber. Subsequently, 5 L/min nitrogen gas was flowed through the electric furnace chamber to replace with a nitrogen atmosphere. The pyrolysis reaction (first decomposition step) was performed by heating the electric furnace to the temperatures listed in Table 1 and holding for the times listed in Table 1 while the same flow rate of nitrogen gas was maintained.

### (Solvent decomposition step)

To 20 mg of the heat-treated rubber obtained as described above, 2 g of heavy chloroform and 0.10 g of precisely weighed hexamethylene disilazane were added as solvent, dispersed by ultrasonic waves, then solid-liquid separated by centrifugation into supernatant liquid and solid component.

The amount of polymer component remaining in the solid content is determined by TGA as the weight loss from 300°C to 650°C by heating in nitrogen from 50°C to 700°C. The decomposition yield of the polymer component was determined by subtracting the amount of polymer component remaining in the solid content from the weight of the polymer component in the rubber.

The supernatant solution was measured by ¹H-NMR, and the yield of isoprene skeleton components in the decomposed polymer component was determined from the hydrogen content at the α-position of the isoprene structure relative to the hydrogen content of hexamethylene disilazane, and is listed in Table 1.

### <Second decomposition step>

The yields of isoprene and limonene were estimated from the yields of isoprene skeleton components obtained in Examples 6 to 13, based on the yields of isoprene and limonene in Example 2 when implemented as per Example 2. The results are summarized in Table 1.

**[Table 1]**

| | | Example 6 | Example 7 | Example 8 | Example 9 | Example 10 | Example 11 | Example 12 | Example 13 |
|---|---|---|---|---|---|---|---|---|---|
| First decomposition step | Electric furnace heating temp. (°C) | 300 | 300 | 270 | 330 | 200 | 200 | 250 | 150 |
| | Electric furnace heating time (h) | 1 | 3 | 1 | 1 | 1 | 3 | 1 | 3 |
| Solvent decomposition step | Decomposition yield of polymer components (mass%) | 59 | 61 | 33 | 52 | 15 | 16 | 32 | 3 |
| | Yield of isoprene skeleton component in polymer component (%) | 58 | 44 | 83 | 35 | 76 | 80 | 81 | 33 |
| Second decomposition step | Isoprene + limonene yield (mass%) | 20 | 15 | 29 | 11 | 26 | 27 | 28 | 11 |

## Claims

1. A method of decomposing a crosslinked rubber, the method comprising:
a first decomposition step of pyrolyzing a crosslinked rubber containing a diene rubber at a temperature of 150°C or more and 400°C or less; and
a second decomposition step of pyrolyzing a decomposition product obtained by the first decomposition step under an inert gas atmosphere and in the presence of a catalyst at a temperature of 300°C or more and 950°C or less.

2. The method of decomposing a crosslinked rubber according to claim 1, wherein 80 mass% or more of the diene rubber in the crosslinked rubber is decomposed to diene oligomers having a weight-average molecular weight of 100 to 50,000 via the first decomposition step.

3. The method of decomposing a crosslinked rubber according to claim 2, wherein 30 mass% or more of the diene oligomers are decomposed to hydrocarbons having 12 or less carbon atoms via the second decomposition step.

4. The method of decomposing a crosslinked rubber according to any one of claims 1 to 3, wherein 20 mass% or more of the diene rubber in the crosslinked rubber is decomposed to hydrocarbons having 12 or less carbon atoms via the first decomposition step and the second decomposition step.

5. The method of decomposing a crosslinked rubber according to any one of claims 1 to 4, wherein the diene rubber includes at least one rubber selected from the group consisting of isoprene skeleton rubber, styrene-butadiene rubber, and butadiene rubber.

6. The method of decomposing a crosslinked rubber according to any one of claims 1 to 5, wherein the second decomposition step is performed in the presence of at least one basic catalyst selected from the group consisting of TiO₂, ZrO₂, MgO, La₂O₃, CeO₂, Y₂O₃, Li₂CO₃, Na₂CO₃, Rb₂CO₃, and Cs₂CO₃.

7. The method of decomposing a crosslinked rubber according to any one of claims 1 to 6, wherein the crosslinked rubber further contains carbon black.

8. The method of decomposing a crosslinked rubber according to any one of claims 1 to 7, wherein the crosslinked rubber further contains sulfur.
